# EUROPEAN PATENT SPECIFICATION

(11) **EP 1 673 365 B1**
(45) Date of publication and mention of the grant of the patent: **12.03.2008**
(21) Application number: 04769498.9
(22) Date of filing: 29.09.2004
(51) Int. Cl.: C07D 335/12

(54) **PROCESS FOR THE PREPARATION OF Z-FLUPENTIXOL**
VERFAHREN ZUR HERSTELLUNG VON Z-FLUPENTIXOL
PROCEDE DE PREPARATION DE Z-FLUPENTIXOL

(30) Priority: 17.10.2003 IT MI20032024
(43) Date of publication of application: 28.06.2006
(73) Proprietor: LABORATORIO CHIMICO INTERNAZIONALE S.p.A., 20122 Milano (IT)
(72) Inventor: VILLANI, Flavio, Lab. Chimico Internazionale S.p.a, I-20122 Milano (IT); NARDI, Antonio, Lab. Chimico Internazionale S.p.a, I-20122 Milano (IT); SALVI, Annibale, Lab. Chimico InternazionaleS.p.a, I-20122 Milano (IT); MAIORANA, Stefano, Lab. Chim. Internazionale S.p.a, I-20122 Milano (IT)
(74) Representative: Trupiano, Federica
(86) International application number: PCT/IB2004/003155
(87) International publication number: WO 2005/037820

(56) References cited:
- GB-A- 925 538
- US-A- 3 282 930
- US-A- 3 681 346

## Description

The present invention relates to a process for the separation of isomers of flupentixol, particularly a process for the preparation of Z-flupentixol and its decanoate ester and novel synthetic intermediates thereof.

### TECHNICAL BACKGROUND

Flupentixol, or 2-[4-[3-[2-trifluoromethyl-thioxanthen-9-ylidene]-propyl]-piperazin-1-yl]-ethanol represents the active ingredient of a neuroleptic drug for the treatment of psychotic disorders, particularly for the treatment of schizophrenia.

Flupentixol consists of a mixture of two geometric isomers, Z and E. The Z isomer of flupentixol, hereinafter also referred to as Z-flupentixol, is the more active isomer and currently also marketed as the decanoate ester thereof.

GB 925538 generically describes that the separation of the two flupentixol isomers can be carried out by the fractional crystallisation of the dihydrochloride. However, such separation seems to have never found a real industrial application.

US 3681346 describes the separation of the Z and E isomers of flupentixol by the fractional crystallisation of flupentixol base from ethyl ether. However, the separation of the two isomers by the crystallisation of flupentixol base is known to provide no good results, above all if there are many impurities present in the starting mixture.

US 3 282 930 describes a process for fractionally crystallising 10-{3-[4-(3-benzoyloxypropyl)-1-piperazinyl] propylidene}-2-trifluoromethylthiaxanthene dihydrochloride to obtain the separate cis and trans isomers (example 3).

### SUMMARY

An object of the present invention is to provide an improved process for the separation of the flupentixol isomers, more precisely a process for the preparation of Z-flupentixol and the decanoate ester thereof, in the pure form.

It has been surprisingly found that the separation of the isomers by the crystallisation of one particular ester of flupentixol provides optimal results in terms of yield and purity.

Indeed, it has been found that one particular ester derivative of flupentixol constitutes an intermediate which is particularly suitable for fractional crystallisation, said ester derivative being easily separable into the two Z and E isomers.

### DETAILED DESCRIPTION OF THE INVENTION

Thus according to one of its embodiments, the present invention concerns a process for the preparation of Z-flupentixol which comprises preparing a hydrochloride of the p-chlorobenzoate ester of flupentixol and separating said ester into its Z and E isomers by fractional crystallisation.

As used in the description herein, even when not expressly indicated, the term "fluxopentixol" refers to a mixture of the Z and E isomers, whilst the terms "E-fluxopentixol" and "Z-fluxopentixol" refer to the pure isomers or to a mixture of the two compounds wherein, the E isomer and the Z isomer are the preponderant isomers. The degree of purity of the mixture is given as the percentage (by weight) of the amount of the preponderant isomer in the mixture.

More particularly, the invention concerns a process for the preparation of Z-flupentixol which comprises:
(a) making Z/E flupentixol react with the chloride salt of p-chlorobenzoic acid in a solvent selected from ethyl acetate, acetone, methyl ethyl ketone, methylene chloride, dioxane and tetrahydrofuran, by heating;
(b) cooling the reaction mixture and separating the precipitate from the mother liquors containing the Z-flupentixol p-chlorobenzoate ester;
(c) heating the mother liquors from the previous step and adding hydrochloric acid;
(d) cooling the reaction mixture in order to recover the hydrochloride salt of the Z-flupentixol p-chlorobenzoate ester thus precipitated;
(e) hydrolysing the ester in order to obtain Z-flupentixol.

The starting flupentixol used in step (a) may be produced by the condensation of 2-trifluoromethyl-9-(-propyliden)-thioxanthene with N-(2-hydroxyethyl)piperazine or prepared using other synthetic methods. Illustrative examples are provided in the experimental section which follows.

Even if crude, the flupentixol directly derived from the aforesaid condensation, which normally requires further purification, may anyway be used in step (a) of the process. Indeed, it has been observed that in the esterification reaction of step (a), notwithstanding the possible presence of impurities in the starting product, the p-chlorobenzoate ester forms in a satisfactory manner and the separation of its isomers by crystallisation proceeds excellently, unlike the same separation carried out directly on crude, non-esterified flupentixol.

According to the present invention, a particularly advantageous solvent for the esterification step (a) is ethyl acetate, in that it allows for excellent separation of the isomers.

In the reaction in step (a), by the expression "by heating" is meant that all the reagents must be brought into solution by heating; preferably, the reaction in step (a) is carried out at a temperature comprised of between 40°C and the reflux temperature of the solvent used, advantageously around 70°C.

The reaction is complete in a few hours, and its progress may be followed by one skilled in the art, using conventional methods.

With the reaction in step (b), the E isomer of the ester thus formed precipitates, whilst the desired Z isomer remains in solution. The precipitate is separated from the mother liquors and the isolated E isomer may be recovered and, if desired, converted according to known techniques. Examples of the recovery of such isomer are reported in the following experimental section.

In step (c), the mother liquors separated in step (b) are lightly heated, for example to between 40 and 50°C, and hydrochloric acid is added therein.

Advantageously, in step (c) hydrochloric acid is added in molar amounts almost equal to half that of the starting Z/E flupentixol, for example, in amounts comprised of between 0.4 and 0.6 moles of hydrochloric acid per mole of starting Z/E flupentixol.

The hydrochloric acid used in step (c) may be for example in the form of an aqueous solution of known concentration.

According to the present invention, the hydrochloride salts of flupentixol or the esters thereof, obtained according to the processes of the invention, may be formed with n equivalents of hydrochloric acid, where n is a number, not necessarily an integer, comprised of between 0.5 and 2. For example hemi-hydrochloride, monohydrochloride, di-hydrochloride salts, etc. may thus be formed, the stoichiometric ratios being a function of the reaction conditions.

Consequently, according to the present invention, unless there is any specific indication to the contrary, by the term "hydrochloride" is meant a salt with hydrochloric acid in any ratio of acid-base equivalents.

Flupentixol p-chlorobenzoate, its Z isomer and salts thereof, including the hydrochloride salts thereof in whichever stoichiometric ratio of acid-base equivalents, are novel products and constitute a further subject of the present invention.

The Z-flupentixol p-chlorobenzoate ester isolated as the hydrochloride in step (d) is normally appreciably pure and may be converted into Z-flupentixol by the hydrolysis in step (e) according to conventional techniques, for example by reacting with a mineral or organic base, with alkaline metal or alkaline earth metal hydroxides, for example with potassium hydroxide.

As mentioned above, the Z and E isomers of flupentixol can be separated according to the process of the present invention, even by means of an ester other than p-chlorobenzoate, for example by using the above mentioned esters, the p-chlorobenzoate ester however being a particularly preferred ester.

If desired or necessary, the Z-flupentixol obtained from step (e) may be further purified by crystallisation from an appropriate solvent.

Particularly, it has now been found that the crystallisation of Z-flupentixol from cyclohexane leads to a particularly pure product, suitable for use as a drug,

The crystallisation of Z-flupentixol from cyclohexane constitutes a further subject of the invention.

According to another aspect thereof, the invention also concerns the process of steps (a) to (e) as defined above wherein, following step (e), the Z-flupentixol obtained is crystallised from cyclohexane.

The crystallisation from cyclohexane is carried out according to conventional techniques, for example by bringing the Z-flupentixol into solution by heating and slowly cooling, in order to obtain the precipitation of the desired product.

The Z-flupentixol obtained following crystallisation from cyclohexane shows purity greater than 99% and conforms to European Pharmacopoeia requirements for this product.

If desired or necessary, the pure Z-flupentixol thus obtained may be later converted into the decanoate ester thereof by simple esterification according to known techniques.

Thus, according to a further aspect, the present invention has as a subject, a process for the preparation of Z-flupentixol decanoate ester which comprises the above described steps from (a) to (e), followed by the following steps:
(f) crystallising the Z-flupentixol from step (e) from cyclohexane; and
(g) converting the pure Z-flupentixol thus obtained into Z-flupentixol decanoate ester.

The crystallisation in step (f) may be carried out as described above.

The esterification reaction in step (g) may be carried out according to the usual techniques well known to any expert in the art, for example, by reaction of the product isolated in step (f) with decanoic acid or an activated derivative thereof, such as the corresponding acyl chloride, in an appropriate solvent.

If the reaction in step (g) is carried out with decanoyl chloride, then a hydrochloride salt of the Z-flupentixol decanoate ester is obtained.

Hence, the invention provides an original and effective process for the separation of the flupentixol isomers and for the preparation of Z-flupentixol decanoate ester, with optimal yield and high purity.

The examples reported in the following experimental section illustrate particular embodiments of the invention, without however being limiting in any way.

Experimental section

### Step 1: preparation of 2-trifluoromethyl-9-allyl-9-thioxanthenol

To a solution of 6.6 g (0.046 mol) of methyl iodide and 0.3 g (0.001 mol) of iodine in 1050 ml of tetrahydrofuran 136.5 g (5.6 mol) of magnesium shavings are added, under an atmosphere of nitrogen. To the suspension thus obtained a solution of 172.5 g (2.25 mol) of allyl chloride in 172.5 ml of tetrahydrofuran is added by following these methods: approx. 10% of the solution is added and the spontaneous initiation of the reaction, indicated by a sudden increase in the temperature, is awaited; whilst maintaining the temperature between 45 and 50°C, the remainder of the solution is then added over a period of approx. 90 minutes. Upon complete addition, the temperature is maintained at 45-50°C for 30 minutes. The reaction mixture is cooled to 25°C and a solution of 315 g (1.12 mol) of 2-trifluoromethyl-9-thioxanthone in 1,425 ml of tetrahydrofuran is added over a period of 2 hours whilst maintaining the temperature below 30°C. At the end of the addition, it is kept stirring for a further hour. The reaction mixture is then poured into a solution of 160 g of ammonium chloride in 1,500 ml of water whilst maintaining the temperature below 30°C. The phases are separated and the aqueous phase is extracted with 750 ml of toluene. The organic phases are combined and washed, first with a solution of 80 g of ammonium chloride in 750 ml of water, and subsequently with a solution of 40 g of sodium chloride in 750 ml of water. The organic phase is concentrated to a residue by the evaporation of the solvent under vacuum and 372 g of 2-trifluoromethyl-9-allyl-9-thioxanthenol (quantitative yield) are obtained. An aliquot of the product has been purified by column chromatography and has been characterised by ¹H-NMR spectroscopic analysis: ¹H-NMR (300 MHz, CDCl₃): 2.52 (1 H, dd); 2.64 (1H, dd); 3 (1H, s); 5.1 (2H, m); 5.42 (1H, m); 7.2-7.8 (7H, m).

### Step 2: preparation of 2-trifluoromethyl-9-(2-propenyliden)-thioxanthene

To a solution of 372 g (1.12 mol) of 2-trifluoromethyl-9-allyl-9-thioxanthenol in 372 ml of toluene, maintained at 40°C, a solution of 5.3 g (0.067 mol) of acetyl chloride in 160.5 g (1.57 mol) of acetic anhydride is added, over a period of 30 minutes. The solution is heated to 50-55°C and maintained at that temperature for one hour. The mixture is concentrated to a residue by evaporation of the solvent under vacuum and 350 g of 2-trifluoromethyl-9-(2-propenyliden)-thioxanthene (quantitative yield) are obtained. An aliquot of the product has been purified by column chromatography and has been characterised by ¹H-NMR spectroscopic analysis: ¹H-NMR (300 MHz, CDCl₃): 5.4 (1 H, m); 5.6 (1 H, m);6.57 (1H, t); 6.83 (1 H, m); 2-7.8 (7H, m)

### Step 3: preparation of 9-[3-(4-hydroxyethyl-1-piperazinyl)propylidene]-2-trifluoromethyl-thioxanthene (Z/E), Z/E Flupentixol

A solution of 350 g (1.12 mol) of 2-trifluoromethyl-9-(2-propenyliden)-thioxanthene in 1,890 g (14.5 mol) of N-(2-hydroxyethyl)piperazine is heated to 100°C for 7 hours. The excess N-(2-hydroxyethyl) piperazine is distilled off at a temperature of 100-120°C under reduced pressure of 0.2-1 mm/Hg. To the residue 2,490 ml of toluene and 490 ml of water are added and it is left stirring for 15 minutes at 70°C. The aqueous phase is separated and two washes of the organic phase are carried out using 490 ml of water (for each wash) whilst maintaining the temperature at 60-70°C. An extraction of the aqueous phase is carried out using 490 ml of toluene and the two organic phases are combined. The combined organic phase is concentrated to a residue by evaporation of the solvent under vacuum. The residue is dissolved in 1,920 ml of ethyl acetate and the solution heated to 50°C. 1,340 ml of 10% aqueous sulphuric acid are added over a period of 15-20 minutes and the mixture is left stirring for 15 minutes. The phases are separated and 920 ml of ethyl acetate added to the acidic aqueous phase. The mixture is left stirring at 60°C for 15 minutes and the phases separated. To the acidic aqueous phase are added 1,920 ml of ethyl acetate, and 190 g of aqueous 30% ammonia are added dropwise whilst maintaining the temperature below 30°C. The phases are separated and the organic phase washed with 900 ml of a 5% aqueous sodium chloride solution. The organic phase is concentrated to residue by the evaporation of the solvent under reduced pressure and 425 g of (Z/E) 9-[3-(4-hydroxyethyl-1-piperazinyl)propylidene]-2-trifluoromethyl-thioxanthene (Flupentixol Z/E) are obtained, yield 87%, isomeric ratio Z/E = 47/53.

### Step 4: preparation of Z Flupentixol p-chlorobenzoate.2HCl

A solution of 425 g (0.98 mol) of Z/E Flupentixol in 1,750 ml of ethyl acetate is heated to 40°C. A solution of 213 g (1.21 mol) of p-chlorobenzoyl chloride in 660 ml of ethyl acetate is added dropwise, the reaction mixture is heated to 70°C and maintained at that temperature for 1 hour. The reaction mixture is cooled to 5°C and the product is filtered, washing it on the filter with 220 ml of ethyl acetate. The wet product is dried at 50°C under vacuum for 24 hours and 332 g of E Flupentixol p-chlorobenzoate.HCl are obtained which is set aside for later recovery. The mother liquors from the previous filtration are heated to 40°C. 43 g (0.43 mol) of 37% aqueous hydrochloric acid are added over 15 minutes and the mixture progressively cooled down to 15°C. The product is filtered, washing it on the filter with 140 ml of ethyl acetate. The wet product is dried at 50°C under vacuum for 24 hours and 208 g of Z Flupentixol p-chlorobenzoate.2HCl are obtained, having an isomeric purity of 85% (15 % E), with a yield of 33%. The product has been characterised by ¹H-NMR spectroscopic analysis: ¹H-NMR (300 MHz, DMSO-d6): 2.79 (2H, m); 3.2-3.7 (12 H, m); 4.53 (2 H, bs); 6.03 (1 H, t), 7.2-8.1 (11 H, m).
MASS SPECTROMETRY (FAB +): 573 (molecular peak).

### Step 5: preparation of Z Flupentixol

To a mixture of 178 g (0.27 mol) of Z Flupentixol p-chlorobenzoate.2HCl, 600 ml of methyl alcohol and 92 ml of water 63 g (1 mol) of 90% potassium hydroxide are added. The mixture is heated to 55°C and maintained at that temperature for 1 hour. It is concentrated to a residue by the distillation of the solvent under reduced pressure and 920 ml of water and 920 ml of toluene are added. The mixture is heated to 70 °C, the phases are separated, and the aqueous phase extracted with 150 ml of toluene. The organic phases are combined and two washes are performed using 300 ml (for each wash) of a 10% aqueous solution of sodium chloride. The organic phase is concentrated to a residue by the evaporation of the solvent under vacuum and 300 ml of cyclohexane are added. It is once again concentrated to a residue and 820 ml of cyclohexane added. The entire mixture is brought into solution by heating to 60°C and the solution filtered whilst maintaining said heating. The solution is cooled slowly to 15°C thus obtaining the crystallisation of the product. The product is filtered and washed on the filter with 70 ml of cyclohexane. The wet product is dried at 40°C under vacuum for 16 hours and 82 g of Z Flupentixol are obtained, having an isomeric purity of 99.7%. Yield 70%. The product has been characterised by ¹H-NMR spectroscopic analysis: ¹H-NMR (300 MHz, CDCl₃): 2.5-2.6 (14H, m); 3.57-3.61 (2 H, t); 5.94-5.97 (1 H, t) 7.22-7.65 (7H, m).
MASS SPECTROMETRY (El +): 434 (molecular peak), 433 (M-H), 415 (M-F), 403 (M-CH₂OH).

### Step 6: preparation of Z Flupentixol decanoate.2HCl

To a solution of 90 g (0.207 mol) of Z Flupentixol in 270 ml of acetone 47.4 g (0.24 mol) of decanoyl chloride are added over a period of 30 minutes. The mixture is heated to reflux temperature (58°C) and maintained at such temperature for 1 hour. The mixture is cooled to 25°C and 1,090 ml of ethyl acetate added. 126 ml of a 1.95 M solution of hydrochloric acid in ethyl acetate are added dropwise thus achieving the precipitation of the product. The reaction mixture is progressively cooled to 5°C and the product is filtered, washing it on the filter with 100 ml of ethyl acetate. The wet product is dried at 50°C under vacuum for 16 hours and 117 g of Z Flupentixol decanoate.2HCl are obtained (yield 85%). The product has been characterised by ¹H-NMR spectroscopic analysis: ¹H-NMR (300 MHz, CDCl₃): 0.85 (3H, t); 1.23 (12H, m);1.56 (2H, m); 2.32 (2H, t); 3 (2H, m) ; 3.2-4.1 (12H, m); 4.52 (2 H, m); 5.9 (1 H, t); 7.2-7.6 (7H, m).
MASS SPECTROMETRY (FAB +): 589 (molecular peak)

### Step 7: preparation of Z Flupentixol decanoate

To a suspension of 75 g (0.113 mol) of Z Flupentixol decanoate.2HCl in 450 ml of tert-Butyl methyl ether, is added dropwise, over a period of 20 minutes, a solution of 18 g (0.13 mol) of potassium carbonate in 200 ml of water. The phases are separated and the organic phase washed with 100 ml of water.

The organic phase is dried over 30 g of anhydrous sodium sulphate and concentrated to a residue by evaporation of the solvent at 40°C under a reduced pressure of 0.1-0.5 mm/Hg. 63 g of Z Flupentixol decanoate are obtained (yield 94%) as a yellow coloured viscous oil. The product has been characterised by ¹H-NMR spectroscopic analysis: ¹H-NMR (300 MHz, CDCl₃): 0.89 (3H, t); 1.23 (12H, m);1.56 (2H, m); 2.32 (2H, t); 2.5 (2H, m) ; 4.2-4.1 (12H, m); 4.52 (2 H, m); 5.9 (1 H, t); 67.2 (7H, m).
MASS SPECTROMETRY (FAB +): 588 (molecular peak), 569 (M-F).

### Recovery of the E Flupentixol p-chlorobenzoate.HCl set aside during step 4

### 1 st step: preparation of E Flupentixol

To a mixture of 1,200 g (1.97 mol) of E Flupentixol p-chlorobenzoate.HCl, 4,200 ml of methyl alcohol and 590 ml of water 364 g (5.8 mol) of 90% potassium hydroxide are added. The mixture is heated to 55°C and maintained at that temperature for 1 hour. The mixture is concentrated to a residue by the distillation of the solvent under reduced pressure and 4,000 ml of water and 4,000 ml of toluene are added. The mixture is heated to 70 °C, the phases are separated, and the aqueous phase extracted with 800 ml of toluene. The organic phases are combined and two washes are performed using 300 ml (for each wash) of a 10% aqueous solution of sodium chloride. The organic phase is concentrated to a residue by evaporation of the solvent under vacuum and 785 g of E Flupentixol E are obtained (yield 91.7 %) as a dense oil.

### 2nd step: preparation of the recovered Z/E Flupentixol.

A solution of 70 g (0.161 mol) of E Flupentixol in 1,400 ml of ethyl acetate is irradiated using a 500 W ultraviolet lamp (having a wavelength emission range between 200 and 300 nm) for 6 hours at 35°C. The solution is concentrated to a residue by evaporation of the solvent under vacuum and 70 g of Z/E Flupentixol are obtained (quantitative yield) having a Z/E isomeric ratio equal to 45/55.

The product thus obtained may be recycled into Step 4 of the synthetic process.

### Preparation of Z/E FLUPENTIXOL DIHYDROCHLORIDE

### The preparation process is as described in Steps 1 to 4 for Z Flupentixol Decanoate

### Step 8: preparation of E Flupentixol di-hydrochloride

To a mixture of 293 g (0.48 mol) of E Flupentixol p-chlorobenzoate.HCl, (89% E, 11 % Z), 1,000 ml of methyl alcohol and 155 ml of water 106 g (1.7 mol) of 90% potassium hydroxide are added. The mixture is heated to 55°C and maintained at that temperature for 1 hour. The mixture is concentrated to a residue by the distillation of the solvent under reduced pressure and 1,535 ml of water and 1,535 ml of toluene are added. The mixture is heated to 70 °C, the phases are separated, and the aqueous phase extracted with 250 ml of toluene. The organic phases are combined and two washes are performed using 500 ml (for each wash) of a 10% aqueous solution of sodium chloride. The organic phase is concentrated to a residue by distillation of the solvent under reduced pressure and 2,080 ml of acetone are added. The residue is heated to 35°C and 98 g (0.99 mol) of aqueous hydrochloric acid are added dropwise over a period of 15 minutes. 37%. The mixture is kept stirring whilst spontaneously cooling over a period of 16 hours. The mixture is cooled to 10°C and filtered using a büchner funnel, washing the filtrate on the filter with 100 ml of acetone. The filtrate is dried at 50°C for 18 hours and 180 g of E Flupentixol di-hydrochloride are obtained (98.1% E, 1.9% Z), yield 74%.

### Step 9: preparation of Z Flupentixol di-hydrochloride

To a mixture of 250 g (0.39 mol) of Z Flupentixol p-chlorobenzoate.2HCl, (75% Z, 25% E), 860 ml of methyl alcohol and 133 ml of water 91 g (1.46 mol) of 90% potassium hydroxide are added. The mixture is heated to 55°C and maintained at that temperature for 1 hour. The mixture is concentrated to a residue by the distillation of the solvent under reduced pressure and 1,315 ml of water and 1,315 ml of toluene are added. The mixture is heated to 70 °C, the phases are separated, and the aqueous phase extracted with 215 ml of toluene. The organic phases are combined and two washes are performed using 500 ml (for each wash) of a 10% aqueous solution of sodium chloride. The organic phases are combined and two washes are performed using 430 ml (for each wash) of a 10% aqueous solution of sodium chloride. The organic phase is concentrated to a residue by distillation of the solvent under reduced pressure and 1,720 ml of acetone are added. The residue is heated to 35°C and 81 g (0.82 mol) of aqueous hydrochloric acid are added dropwise over a period of 15 minutes. 37%. The mixture is kept stirring whilst spontaneously cooling over a period of 16 hours. The mixture is cooled to 10°C and filtered using a büchner funnel, washing the filtrate on the filter with 100 ml of acetone. The filtrate is dried at 50°C for 18 hours and 146 g of Z Flupentixol di-hydrochloride are obtained (74% Z, 26% E), yield 74%.

### Step 10: preparation of Z/E Flupentixol di-hydrochloride.

To a mixture of 1,900 ml of isopropyl alcohol and 64 ml of distilled water 75 g of E Flupentixol di-hydrochloride (98.1 % E, 1.9% Z) and 140 g of Z Flupentixol di-hydrochloride (74% Z, 26% E) are added. The amounts of the two di-hydrochloride salts used depend on the Z/E isomeric ratios of the individual products: the calculation is performed in such a way as to obtain, in the initial crystallisation solution, a Z isomer content of 47-52% (in this case, it is equal to 48.8%). The mixture is heated to reflux temperature (80°C) thus obtaining complete dissolution. 5 g of activated charcoal are added and the solution filtered whilst maintaining the temperature above 50°C. The solution is progressively cooled thus achieving the crystallisation of the product. The solution is brought to a temperature of 5°C and filtered using a büchner funnel, washing the filtrate with 200 ml of isopropyl alcohol. The wet product is dried at 50°C under vacuum for 24 hours thus obtaining 185.5 g (yield 86%) of Z/E FLUPENTIXOL DIHYDROCHLORIDE (45.5% Z, 54.5% E)

The product has been characterised by ¹H-NMR spectroscopic analysis: ¹H-NMR (300 MHz, CDCl₃): 2.62 (2H, m); 2.9-3.2 (2 H, m); 3.37 (6 H, m); 3.59 (4H, m); 3.9 (2H m); 5.48 (1H, t, E isomer); 5.68 (1H, t, Z isomer); 6.8-7.5 (7H, m).

## Claims

1. A process for the preparation of Z-flupentixol **characterised by** preparing a hydrochloride of the p-chlorobenzoate ester of flupentixol and separating the two Z and E isomers of said ester by fractional crystallisation.

2. The process according to claim 1, **characterised by** comprising the following steps:
(a) reacting Z/E flupentixol with the p-chlorobenzoic acid chloride in a solvent selected from ethyl acetate, acetone, methyl ethyl ketone, methylene chloride, dioxane and tetrahydrofuran, by heating;
(b) cooling the reaction mixture and separating the precipitate from the mother liquors containing the Z-flupentixol p-chlorobenzoate ester;
(c) heating the mother liquors from the previous step and adding hydrochloric acid;
(d) cooling the reaction mixture in order to recover the hydrochloride salt of the Z-flupentixol p-chlorobenzoate ester thus precipitated; and
(e) hydrolysing the ester in order to obtain Z-flupentixol.

3. The process according to claim 2, **characterised in that** in step (a) said solvent is ethyl acetate.

4. The process according to claim 2, **characterised in that** in step (a) the reaction temperature is comprised of between 40°C and the reflux temperature of the solvent.

5. The process according to claim 4, **characterised in that** said temperature is approx. 70°C.

6. The process according to claim 2, **characterised in that** in step (b) the E-flupentixol is separated by filtration.

7. The process according to claim 2, **characterised in that** in step (c) hydrochloric acid is added in molar amounts equal to approximately half that of the starting flupentixol.

8. The process according to claim 7, **characterised in that** hydrochloric acid is added in amounts of 0.4-0.6 mol of hydrochloric acid per mole of starting flupentixol.

9. The process according to claim 2, **characterised in that** in step (e) hydrolysis is carried out using alkaline metal or alkaline earth metal hydroxides.

10. The process according to claim 2, **characterised in that** following step (e), the Z-flupentixol obtained is crystallised from cyclohexane.

11. The process according to claim 2, **characterised in that** after step (e) the following steps are carried out:
(f) crystallisation of the Z-flupentixol from step (e) from cyclohexane; and
(g) conversion of the pure Z-flupentixol thus obtained into Z-flupentixol decanoate.

12. A compound selected from flupentixol p-chlorobenzoate isomeric mixture, its Z isomer and salts thereof.

13. The compound according to claim 12 which is selected from Z-flupentixol p-chlorobenzoate hemi-hydrochloride, monohydrochloride and di-hydrochloride.

## Patentansprüche

1. Verfahren zur Herstellung von (Z)-Flupentixol, **gekennzeichnet durch** die Darstellung eines Hydrochlorids aus dem p-Chlorbenzoesäureester von Flupentixol und die Trennung der beiden (Z)- und (E)-Isomere des Esters **durch** fraktionierende Kristallisation.

2. Verfahren nach Anspruch 1, **dadurch gekennzeichnet, dass** dieses die folgenden Schritte umfasst:
(a) Umsetzung von (Z)-/(E)-Flupentixol mit dem p-Chlorbenzoesäurechlorid durch Erhitzen in einem Lösungsmittel, ausgewählt unter Ethylacetat, Aceton, Methylethylketon, Methylenchlorid, Dioxan und Tetrahydrofuran;
(b) Kühlen der Reaktionsmischung und Abtrennen des Niederschlags von den Mutterlaugen, welche den (Z)-Flupentixol-p-chlorbenzoesäureester enthalten;
(c) Erhitzen der Mutterlaugen aus dem vorangegangenen Schritt und Zusetzen von Salzsäure;
(d) Kühlen der Reaktionsmischung, um das Hydrochlorid-Salz des so ausgefällten (Z)-Flupentixol-p-chlorbenzoesäureesters zu gewinnen; und
(e) Hydrolysieren des Esters, um (Z)-Flupentixol zu erhalten.

3. Verfahren nach Anspruch 2, **dadurch gekennzeichnet, dass** das Lösungsmittel in Schritt (a) Ethylacetat ist.

4. Verfahren nach Anspruch 2, **dadurch gekennzeichnet, dass** in Schritt (a) die Reaktionstemperatur zwischen 40°C und der Rückflusstemperatur des Lösungsmittels liegt.

5. Verfahren nach Anspruch 4, **dadurch gekennzeichnet, dass** die Temperatur etwa 70°C beträgt.

6. Verfahren nach Anspruch 2, **dadurch gekennzeichnet, dass** in Schritt (b) das (E)-Flupentixol durch Filtration abgetrennt wird.

7. Verfahren nach Anspruch 2, **dadurch gekennzeichnet, dass** in Schritt (c) Salzsäure in molaren Mengen zugesetzt wird, welche ungefähr gleich der Hälfte der Ausgangsstoffmenge an Flupentixol sind.

8. Verfahren nach Anspruch 7, **dadurch gekennzeichnet, dass** Salzsäure in Mengen von 0,4 - 0,6 mol an Salzsäure je Mol der Ausgangsstoffmenge an Flupentixol zugesetzt wird.

9. Verfahren nach Anspruch 2, **dadurch gekennzeichnet, dass** in Schritt (e) das Hydrolysieren ausgeführt wird unter Verwendung von Alkalimetall- oder Erdalkalimetallhydroxiden.

10. Verfahren nach Anspruch 2, **dadurch gekennzeichnet, dass** auf Schritt (e) folgend das erhaltene (Z)-Flupentixol aus Cyclohexan kristallisiert wird.

11. Verfahren nach Anspruch 2, **dadurch gekennzeichnet, dass** nach Schritt (e) die folgenden Schritte ausgeführt werden:
(f) Kristallisation des (Z)-Flupentixol aus Schritt (e) aus Cyclohexan; und
(g) Umwandlung des so erhaltenen reinen (Z)-Flupentixol zu (Z)-Flupentixolde-canoat.

12. Verbindung, ausgewählt unter einem Isomerengemisch von Flupentixol-p-chlorbenzoat-, dessen Z-Isomer und Salzen davon.

13. Verbindung nach Anspruch 12, welche ausgewählt wird unter dem Hemihydrochlorid, dem Monohydrochlorid und dem Dihydrochlorid von (Z)-Flupentixol-p-chlorbenzoat.

## Revendications

1. Procédé de préparation de Z-flupentixol **caractérisé par** la préparation d'un chlorhydrate de p-chlorobenzoate ester de flupentixol et la séparation des deux isomères Z et E dudit ester par cristallisation par fractionnement.

2. Procédé selon la revendication 1, **caractérisé en ce qu'**il comprend les étapes suivantes :
(a) réaction du Z/E flupentixol avec le chlorure d'acide p-chlorobenzoïque dans un solvant choisi parmi l'acétate d'éthyle, l'acétone, la méthyl-éthyl-cétone, le chlorure de méthylène, le dioxane et le tétrahydrofurane, en chauffant ;
(b) refroidissement du mélange réactionnel et séparation du précipité des liqueurs mères contenant le p-chlorobenzoate ester de Z-flupentixol ;
(c) chauffage des liqueurs mères de l'étape précédente et addition d'acide chlorhydrique ;
(d) refroidissement du mélange réactionnel afin de récupérer le sel chlorhydrate de p-chlorobenzoate ester de Z-flupentixol ainsi précipité ; et
(e) hydrolyse de l'ester afin d'obtenir le Z-flupentixol.

3. Procédé selon la revendication 2, **caractérisé en ce qu'**à l'étape (a), ledit solvant est l'acétate d'éthyle.

4. Procédé selon la revendication 2, **caractérisé en ce qu'**à l'étape (a), la température réactionnelle est comprise entre 40° C et la température de reflux du solvant.

5. Procédé selon la revendication 4, **caractérisé en ce que** ladite température est d'environ 70° C.

6. Procédé selon la revendication 2, **caractérisé en ce qu'**à l'étape (b), le E-flupentixol est séparé par filtration.

7. Procédé selon la revendication 2, **caractérisé en ce qu'**à l'étape (c), l'acide chlorhydrique est ajouté en quantités molaires égales à environ la moitié de celle du flupentixol de départ.

8. Procédé selon la revendication 7, **caractérisé en ce que** l'acide chlorhydrique est ajouté en quantités de 0,4 à 0,6 mole d'acide chlorhydrique par mole de flupentixol de départ.

9. Procédé selon la revendication 2, **caractérisé en ce qu'**à l'étape (e), l'hydrolyse est réalisée en utilisant des hydroxydes de métal alcalin ou de métal alcalino-terreux.

10. Procédé selon la revendication 2, **caractérisé en ce qu'**après l'étape (e), le Z-flupentixol obtenu est cristallisé à partir de cyclohexane.

11. Procédé selon la revendication 2, **caractérisé en ce qu'**après l'étape (e), les étapes suivantes sont réalisée :
(f) cristallisation du Z-flupentixol de l'étape (e) à partir de cyclohexane ; et
(g) conversion du Z-flupentixol pur ainsi obtenu en Z-flupentixol décanoate.

12. Composé choisi parmi un mélange isomère de p-chlorobenzoate de flupentixol, son isomère Z et leurs sels.

13. Composé selon la revendication 12 qui est choisi parmi l'hémi-chlorhydrate, le mono-chlorhydrate et le di-chlorhydrate de p-chlorobenzoate de Z-flupentixol.
